(19) Europäisches Patentamt
European Patent Office
Office européen des brevets

(11) **EP 3 716 935 B1**

(12) **EUROPEAN PATENT SPECIFICATION**

(45) Date of publication and mention
of the grant of the patent:
**13.10.2021 Bulletin 2021/41**

(21) Application number: **18796667.6**

(22) Date of filing: **05.11.2018**

(51) Int Cl.:
*A61K 8/25* (2006.01)       *A61Q 1/00* (2006.01)
*A61Q 19/00* (2006.01)       *A61K 8/895* (2006.01)
*A61K 8/02* (2006.01)

(86) International application number:
**PCT/EP2018/080128**

(87) International publication number:
**WO 2019/105685 (06.06.2019 Gazette 2019/23)**

(54) **COSMETIC COMPOSITION FOR BLURRING SURFACE IMPERFECTIONS OF SKIN**

KOSMETISCHE ZUSAMMENSETZUNG ZUR VERSCHLEIERUNG VON OBERFLÄCHENIMPERFEKTIONEN DER HAUT

COMPOSITION COSMÉTIQUE PERMETTANT D'ESTOMPER DES IMPERFECTIONS DE SURFACE DE LA PEAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2017 PCT/CN2017/113805**
**25.01.2018 EP 18153419**

(43) Date of publication of application:
**07.10.2020 Bulletin 2020/41**

(73) Proprietors:
• **Unilever Global IP Limited**
**Wirral, CH62 AZD (GB)**
Designated Contracting States:
**CY DE GB IE IT MT**
• **Unilever IP Holdings B.V.**
**3013 AL Rotterdam (NL)**
Designated Contracting States:
**AL AT BE BG CH CZ DK EE ES FI FR GR HR HU IS LI LT LU LV MC MK NL NO PL PT RO RS SE SI SK SM TR**

(72) Inventors:
• **HU, Qinghong**
**Shanghai 200335 (CN)**
• **WANG, Lin**
**Shanghai 200335 (CN)**
• **ZHU, Shuqi**
**Shanghai 200335 (CN)**

(74) Representative: **James, Helen Sarah**
**Unilever N.V.**
**Unilever Patent Group**
**Bronland 14**
**6708 WH Wageningen (NL)**

(56) References cited:
**WO-A1-2015/138157       WO-A1-2016/152872**
**WO-A1-2017/071886       WO-A1-2017/220310**
**WO-A2-2007/017843**

**Description**

**Field of the invention**

The present invention relates to cosmetic compositions. More particularly, the invention relates to compositions for blurring or hiding surface imperfections of skin.

**Background of the invention**

Ageing affects the appearance of skin and it usually manifests itself as wrinkles, age spots or as generalised uneven skin tone. Shiny skin indicates that the skin is oily and people do not like it when their skin turns oily. People generally like matte or non-oily skin.

Consumers constantly demand newer and more efficacious cosmetic compositions and one of their needs is those compositions which can mask or hide the imperfections of skin. Usually, the formulation scientists rely on talc, silica, kaolin and other inorganic particulate materials which have high refractive indices.

An alternative is to create a blurring effect. In this case, the incident light is distorted by scattering (lensing). Certain ingredients an achieve this effect. Such ingredients operate as lenses and they bend and twist the light in several directions.

WO15166459 A1 (L'Oreal) discloses a skin care/makeup composition containing a microcapsule containing an encapsulated releasable material. The microcapsule comprises a frangible solid core having a two-layered shell surrounding the core. The core is a material having high oil-absorption and optionally, it is porous. The compositions disclosed in the publication are non-gritty.

WO08018046 A2 (P&G) discloses personal care compositions comprising an emulsifying silicone elastomer in aqueous phase. The compositions comprise a core-shell particulate of from about 5 to 100 $\mu$m. The solid core, which is formed of particulate materials like wax, mica, silica, silicates or alumina is coated with silicone. The compositions enhance the appearance and feel of the user's skin.

US2007179241A (L'Oreal) discloses a cosmetic composition containing at least one silicone elastomer and at least one encapsulated pigment. Refractive index of the pigment (TiO2/ZnO) is greater than the refractive index of the encapsulating material (e.g., silica). The composition is recommended for soft focus benefits.

WO15055416 A1 (Unilever) discloses cosmetic compositions in the form of a paste, gel, liquid or monolithic solid and comprising a particle active, wherein the particle active comprises water-insoluble shell component comprising inorganic material and having a refractive index of from 1.3 to 1.8; and core component comprising volatile liquid and having a refractive index of at least 1.2, wherein the core component comprises at least 75% water by weight of the core component; and a cosmetically acceptable carrier comprising hydrophobic material, wherein the hydrophobic material comprises less than 15% liquid oil by weight of the composition; and wherein the composition comprises 0.001 to 3 wt % yellow pigment. The composition provides longer-lasting optical benefits.

WO15055494 A1 (Unilever) discloses a cosmetic composition comprising a particulate active and opacifying particles having refractive index of at least 1.7. The particle active comprises water-insoluble shell comprising inorganic material and having a refractive index of from 1.3 to 1.8. The core comprises a volatile liquid having a refractive index of at least 1.2.

WO2017071886 A1 (Unilever) discloses a personal composition comprising turbostratic boron nitride, porous silica having a specific surface area of higher than 300m$^2$/g and silicone elastomer. The porous silica has the ability of absorbing large amounts of oils. The composition provides both blurring and lightness benefits.

WO2017220310 A1 (Unilever) discloses a personal care composition comprising retinol, porous silica, silicone elastomer. The composition provides more stable retinol over long-term storage and blurring benefit.

WO 2016152872 (Asahi Glass Company) discloses a cosmetic comprising hollow silica particles having an average particle size of 0.1 to 30m, an outer shell thick ness of 5-500nm and an outer shell thickness 0.5-20% of the average particle size. The cosmetic has a high shielding property.

In these two publications mentioned immediately above, an index called as L&W Index is used to compare efficacy of a given cosmetic composition vis-a-vis others. The Index is directly proportional to performance.

Usually, a formulation scientist would use elastomeric silicone particles to increase the Index. However, in cosmetic compositions, there is an obvious limit on the amount of such particles due to their cost and the unwanted secondary effects like instability and the possibility of adversely affecting tactile properties of the composition.

It has been determined that the problem can be solved by combining elastomeric silicone particles with a core-shell particulate active comprising a water-insoluble shell having refractive index of from 1.3 to 1.8 surrounding a core comprising a fluid whose refractive index is at least 1.0.

Usually, the cosmetic compositions meant for blurring the surface imperfections do not provide skin lightening because it is believed that skin lightening and blurring are two distinct and mutually exclusive effects. However, the present inventors have determined that under certain conditions, the compositions of the invention can provide both the effects. Further, insofar as the L&W Index is concerned, the effect does not wear-off as quickly but withstands some sebum on the skin. Therefore, the effect is long-lasting.

**Summary of the invention**

In accordance with a first aspect is disclosed a cosmetic composition comprising:

(i) a particulate material comprising a shell and a core, where said shell comprises a water-insoluble material having refractive index ($n_s$) of 1.2 to 2.0 at 25 °C and wavelength of 589 nm and said core comprises a fluid having refractive index ($n_c$) of at least 1.0 at 25 °C and wavelength of 589 nm and where the oil absorption capacity of said particulate material is at least 300 g/100g measured by ASTM D281-95 by Spatula Rub-out using linseed oil; and,
(ii) a silicone elastomer having average particle diameter of 0.1 to 100 $\mu$m.

wherein ratio of the amount of said particulate material to that of said particulate silicone elastomer is 1:3.5 to 3.5:1 parts by weight.

In a second aspect is disclosed a method of blurring surface imperfections of skin comprising a step of applying thereon a cosmetic composition of the first aspect.

In a third aspect is disclosed use of a cosmetic composition of the first aspect for blurring surface imperfections of skin.

In a fourth aspect is disclosed a method of lightening the tone of skin comprising a step of applying thereon a cosmetic composition of the first aspect.

In a fifth aspect is disclosed use of a cosmetic composition of the first aspect for lightening the tone of skin.

All other aspects of the present invention will more readily become apparent upon considering the detailed description and examples which follow.

**Definitions**

By "a cosmetic composition" as used herein, is meant to include a composition for topical application to the skin of mammals, especially human beings. Such a composition may be generally classified as leave-on or rinse off but is preferably of the leave on type. The composition is formulated into a product which is applied to a human body specifically for improving appearance but may, in addition, also provide cleansing, odor control or general aesthetics. The composition of the present invention can be in the form of a liquid, lotion, cream, foam, scrub, gel, or toner, or applied with an implement or via a face mask or a pad. Non-limiting examples of such compositions include leave-on skin lotions, creams, antiperspirants, deodorants, lipsticks, foundations, mascara, sunless tanners and sunscreen lotions. The composition of the present invention is preferably a leave-on composition. "Skin" as used herein is meant to include skin on the face and body (e.g., neck, chest, back, arms, underarms, hands, legs, buttocks and scalp) and especially to the sun exposed parts thereof.

Refractive Index

Refractive index values referred to herein are determined at 25 °C and wavelength of 589 nm.

Particle Size

Where the size of particles is mentioned (other than "primary particle size") this means the number average diameter determined, for example using scanning electron microscopy (SEM). Similarly, "thickness" means the number average thickness determined, for example using scanning electron microscopy (SEM). In a preferred method, cryo-SEM is used to determine particle size and thickness. In cryo-SEM, a composition is quick-frozen in liquid nitrogen followed by cryo-planing to create a flat surface to be imaged. Typically, the diameter and thickness are each averaged over at least 200 particles. For diameter determination, intact particles are selected from the SEM image whilst for shell thickness, particles which have been sectioned are selected.

If a particle is not spherical, e.g., oval or spheroidal, then "diameter" means the largest distance measurable across the particle.

Where primary particle size is mentioned this means the size (diameter) measurable by transmission electron microscopy (TEM) using a method such as that described by S. Gu et al in Journal of Colloid and Interface Science, 289 (2005) pp. 419-426.

Hydrophobic Material

By "hydrophobic material" is meant a substance that is attracted to and tends to dissolve in a non-polar solvent (such as n-octanol) in preference to water at 25 °C and 1 atm. Preferred hydrophobic materials are insoluble in water. The term "liquid oil" as used herein refers to hydrophobic material with a melting point below 25 °C at 1 atm. "Non-liquid hydrophobic material" refers to hydrophobic material other than liquid oil. Preferred hydrophobic materials are non-volatile.

Solubility

"Soluble" and "insoluble", as used herein, refers to the solubility of a substance in a solvent (which, unless otherwise stated is water). Soluble means a substance that dissolves in the solvent to give a solution with a concentration of at least 1 g per litre at room temperature (25 °C) and pressure (1 atm). Insoluble means a substance that dissolves in the solvent to give a solution with a concentration of less than 1 g per litre at room temperature and pressure.

Volatility

The term "volatile" as used herein refers to a substance which has a vapour pressure of at least 1000 Pa at 25 °C and a non-volatile substance is one wherein the vapour pressure is less than 1000 Pa.

Leave-on and Wash-off

The term "leave-on" as used with reference to compositions herein means a composition that is applied to or rubbed on the skin, and left thereon. Examples include creams and lotions.
The term "wash-off' as used with reference to compositions herein means a skin cleanser that is applied to or rubbed on the skin and rinsed off substantially immediately subsequent to application. An example is body-wash gel.

Miscellaneous

Except in the examples, or where otherwise explicitly indicated, all numbers in this description indicating amounts of material or conditions of reaction, physical properties of materials and/or use may optionally be understood as modified by the word "about".
All amounts are by weight of the final composition, unless otherwise specified.
The term "solid" as used herein means that the material is not fluid at 25 °C.
It should be noted that in specifying any range of values, a given upper value can be associated with any lower value.
For the avoidance of doubt, the word "comprising" is intended to mean "including" but not necessarily "consisting of" or "composed of". In other words, the listed steps or options need not be exhaustive.
The disclosure of the invention as found herein is to be considered to cover all embodiments as found in the claims as being multiply dependent upon each other irrespective of the fact that claims may be found without multiple dependency or redundancy.
Where a feature is disclosed with respect to a particular aspect of the invention (for example a composition of the invention), such disclosure is also to be considered to apply to any other aspect of the invention (for example a method of the invention) *mutatis mutandis.*

**Detailed description**

The particulate material

Cosmetic compositions in accordance with this invention comprise a particulate material. This material comprises a shell and a core.
The core comprises a fluid having refractive index ($n_c$) of at least 1.0. It is preferred that the fluid is air or a volatile liquid or a non-volatile liquid. It is preferred that the non-volatile liquid is an emollient. Alternatively, the core consists essentially of the fluid. Further preferably the core consists of the fluid. A particularly preferred volatile liquid is water. Alternatively, it is preferred that the volatile liquid is a volatile silicone such as D5 or a $C_{1-5}$ alcohol. Alternatively, the liquid is not too volatile, otherwise it may prematurely escape from the core. Preferably the core comprises at least 50 % water, more preferably at least 75%, furthermore preferably at least 85% and most preferably from 90 to 100% water. The liquid is volatile such that it may evaporate from the core when the particulate material is exposed to air at 25 °C and 1 atm. This happens when the composition is applied to the skin. It is preferred that vapour pressure of the volatile liquid is at least 2 kPa at 25 °C, most preferably at least 2.5 kPa. Further preferably the vapour pressure of the volatile liquid is less than 50 kPa at 25 °C, more preferably less than 15 kPa.

It is preferred that the refractive index ($n_c$) of the core is 1.0 to 1.8, more preferably 1.25 to 1.5 and most preferably 1.3 to 1.45. Preferably the fluid is a volatile liquid or a non-volatile liquid. It is preferred that the volatile liquid is water, cyclomethicone or a cosmetically acceptable volatile solvent other than water. Particularly preferred volatile solvent is $C_{2-3}$alcohol. When the fluid is non-volatile it preferably is an emollient. Emollients are used widely in cosmetics for care and protection of the skin. Emollients are the materials used for prevention of or relief from dryness.

Preferably the emollient is a non-hydrocarbon emollient. Hydrocarbon, as used herein, refers to an organic compound consisting entirely of hydrogen and carbon. Preferably the non-hydrocarbon emollient is an alkenyl or alkyl ester of a $C_{10-20}$ fatty acid, an ether-ester, a polyhydric alcohol ester, a wax ester, a mono-, di- or triglyceride, a sterol ester, a fatty alcohol, a fatty acid, lanolin or derivative, wax ester, a phospholipid, beeswax or a sterol.

More preferably the non-hydrocarbon emollient is a triglyceride. Further preferably the triglyceride is one or more of wheatgerm oil, apricot kernel oil, avocado oil, sunflower seed oil, arnica oil, evening primrose oil, jojoba oil, coconut oil, palm kernel oil, groundnut oil, safflower oil, cotton seed oil, rape seed oil, palm oil, almond oil, rice bran oil, cocoa butter or castor oil.

Alternatively, the emollient is a hydrocarbon emollient. Suitable examples include cyclic hydrocarbons, straight chain aliphatic hydrocarbons (saturated or unsaturated), and branched chain aliphatic hydrocarbons (saturated or unsaturated). Illustrative examples include mineral oil, wax and petrolatum (such as petroleum jelly).

When the cosmetic composition is applied to the skin, the fluid from the core escapes. If it is volatile liquid, it evaporates. When the fluid is an emollient, it gets absorbed by the skin and depending on the nature of the emollient, it may happen quickly or over a period. Thereafter, the fluid in the core is replaced by air. When this happens, the refractive index of the shell exceeds the refractive index of the core (air — R.I. is 1)

The shell comprises a water-insoluble material having refractive index ($n_s$) of 1.2 to 2.0. It is preferred that the refractive index ($n_s$) is 1.3 to 1.8. Preferably the water-insoluble material having refractive index of 1.2 to 2.0 is an inorganic material. More preferably it is an inorganic polymer or an inorganic polymer comprising one or more organic groups. Further preferably the inorganic polymer is obtained by a sol-gel process. It is further preferred that the inorganic polymer comprises silica or silica modified with one or more organic groups. Such polymers can be formed into shell-like particles using methods such as those described for example in US7758888 B1. Preferably the shell comprises at least 80% by weight inorganic material, more preferably at least 90% and most preferably from 95 to 100%.

Without wishing to be bound by theory, it is believed that as the fluid, e.g. the volatile liquid, evaporates from the core (e.g. after application to skin), it is replaced by air. Air has comparatively lower refractive index than the shell which leads to a difference in the concerned refractive indices of the core and the shell. In turn, this causes more light to be light scattered by the particles and the overall optical benefits provided by the composition gradually evolve with time after application.

The shell comprises a water-insoluble material but it preferably is permeable to the fluid. Preferably it is permeable enough to allow the fluid to evaporate or be released and absorbed by the skin when the particulate material is exposed to air at 25 °C and 1 atm. Most preferably the shell is permeable enough to allow substantially all (e.g., 90 to 100% by weight of the total fluid in the core) to evaporate or be released from within.

Refractive index of the water insoluble material is 1.2 to 2.0, more preferably 1.3 to 1.8, furthermore preferably 1.4 1.7 and most preferably 1.5 to 1.6. 1.6

It is preferred that ratio of the refractive index of the core to that of the shell ($n_c/n_s$) is 0.7 to 1.2, more preferably from 0.8 to 1.1 and most preferably from 0.85 to 1.0.

It is observed that the particulate material is better able to scatter light if it is micronized. Accordingly, it is preferred that the particle size (D) of the particulate material is 0.1 to 2 $\mu$m, more preferably 0.2 to 1.2 $\mu$m.

Particulate material with thicker shells provide especially good optical effects. Thus, it is preferred that the relationship between the thickness (L) of the shell and the size (D) of the particulate material follows equation (I) given below:

$$L \geq 150 \text{ nm} - 0.25 \times D$$

It is preferred that the particles of said particulate material are spherical or spheroidal, more preferably spherical.

Further preferably, each particle consists or consists essentially of a single core encapsulated by a single shell.

The compositions of the invention comprise 2.5 to 10 wt% of the particulate material, more preferably up to 6 wt% and even more preferably up to 4 wt% by weight of the composition.

The oil absorption capacity of the particulate material is at least 300 g/100g. The bulk oil absorption of the particulate material is measured according to ASTM D281-95 by Spatula Rub-out using linseed oil.

The Particulate Silicone Elastomer

"Silicone elastomer" as used herein refers to deformable organopolysiloxane with viscoelastic properties.

It is preferred that the silicone elastomer is cross-linked. The silicone elastomer can be obtained from curable organo-polysiloxanes. Examples in this respect are: addition reaction-curing organopolysiloxane compositions which cure under platinum metal catalysis by the addition reaction between SiH-containing diorganopolysiloxane and organopolysiloxane having silicon-bonded vinyl groups; condensation-curing organopolysiloxane compositions which cure in the presence of an organotin compound by a dehydrogenation reaction between hydroxyl terminated diorganopolysiloxane and SiH-containing diorganopolysiloxane; condensation-curing organopolysiloxane compositions which cure in the presence of an organotin compound or a titanate ester, by a condensation reaction between a hydroxyl terminated diorganopolysiloxane and a hydrolyzable organosilane (this condensation reaction is exemplified by dehydration, alcohol-liberating, oxime-liberating, amine-liberating, amide-liberating, carboxyl-liberating, and ketone-liberating reactions); peroxide-curing organopolysiloxane compositions which thermally cure in the presence of an organoperoxide catalyst; and organopolysiloxane compositions which are cured by high-energy radiation, such as by gamma-rays, ultraviolet radiation or electron beams. The silicone elastomer is preferably obtained by addition reaction-curing organopolysiloxane compositions which cure under platinum metal catalysis by the addition reaction between SiH-containing diorganopolysiloxane and organopolysiloxane having silicon-bonded vinyl groups

The silicone elastomer may either be an emulsifying or non-emulsifying cross-linked silicone elastomer or a combination thereof but preferably the silicone elastomer is non-emulsifying. The term "non-emulsifying," as used herein, defines cross-linked silicone elastomer from which poly-oxyalkylene units are absent. The term "emulsifying," as used herein, means cross-linked organo-polysiloxane elastomer having at least one poly-oxyalkylene (e.g., poly-oxyethylene or poly-oxypropylene) unit.

Preferred silicone elastomers are organo-polysiloxanes available under the INCI names of dimethicone/vinyl dimethicone crosspolymer, dimethicone crosspolymer and Polysilicone-11. More preferably the silicone elastomer is dimethicone/vinyl dimethicone crosspolymer.

A commonly available and suitable elastomer is DC9509 (63% solids content) Dimethicone/Vinyl dimethicone Crosspolymer (and) C12-14 Pareth-12. DOW CORNING 9509 Silicone Elastomer Suspension is a 63% nonionic suspension of the spherical silicone elastomer powder in water. Representative of this material are dimethicone/vinyl dimethicone crosspolymers and dimethicone crosspolymers available from a variety of suppliers including Dow Corning (9040, 9041, 9045, 9506 and 9509), General Electric (SFE 839), Shin Etsu (KSG-15, 16 and 18 [dimethicone/phenyl vinyl dimethicone crosspolymer]), and Grant Industries (Gransil brand of materials), and lauryl dimethicone/vinyl dimethicone crosspolymers supplied by Shin Etsu (e.g. KSG-31, KSG-32, KSG-41, KSG-42, KSG-43 and KSG-44). The reference to solids content indicates that when e.g., 10 g of the material is dosed into a given formulation, the effective amount of elastomer being dosed is 6.3 g.

The average particle diameter of the silicone elastomer is 0.1 to 100 $\mu$m. For example, the average particle diameter of silicone elastomer DC9509® from Dow Corning is the particle size D50 tested by Horiba LA-500. It is preferred that particles of the silicone elastomer are spherical or spheroidal. Preferably, the compositions of the invention comprise 0.1 to 10 wt% of the silicone elastomer, more preferably 0.5 to 8 wt%, even more preferably 1 to 5 wt%.

In the compositions according to the invention, the ratio of the amount of the particulate material to that of the particulate silicone elastomer is 1:3.5 to 3.5:1 parts by weight. For further clarity, it is clarified that reference to the amount of silicone elastomer is made on the actual/effective amount thereof without considering the solvents or other carrier ingredients that are usually present in commercially available silicone elastomers.

The ratio enables the formulation of a cosmetic compositions with tunable whitening efficacy which, at the discretion of the formulation scientist, could be increased or decreased by adjusting the ratio of the two ingredients to get whitening effect or no (or lesser) whitening effect, depending on the said ratio. In addition, the compositions also provide L&W Index as indicated/defined earlier, which signifies the ability of the compositions to blur/hide surface imperfections of skin.

Other Ingredients

The composition of the present invention may additionally comprise opacifying particles. These are particles with very high refractive index, i.e., having a refractive index of at least 1.7. For example, the opacifying particles may have refractive index greater than 1.8, more preferably greater than 1.9 and most preferably from 2.0 to 2.7. Examples of such opacifying particles are those comprising bismuth oxy-chloride, boron nitride, titanium dioxide, zirconium oxide, aluminium oxide, zinc oxide or combinations thereof. More preferred are opacifying particles comprising zinc oxide, zirconium oxide, titanium dioxide or a combination thereof as these materials have especially high refractive index. Most preferred are opacifying particles comprising titanium dioxide. Preferably the composition comprises 0.001 to 10 wt%, more preferably 0.01 to 7 wt% opacifying particles.

It is preferred that, when present, the opacifying particles are micronized. Preferably the primary particle size is 20 to 2000 nm, more preferably 25 to 900 nm and most preferably f30 to 400 nm.

The opacifying particles are not the same as the particulate material. Owing to their greater opacifying ability, the opacifying particles are typically compounded in the composition at a lower level.

In addition, the compositions of the invention may also comprise a yellow pigment to provide appearance benefits to skin. Sometimes, the opacity afforded to the skin by the particulate active may appear as uneven "patches" and the yellow pigment can ameliorate this problem.

Preferably the compositions comprise 0.001 to 3 wt% of the yellow pigment, more preferably 0.01 to 2.5 wt%, more preferably still from 0.05 to 2 wt%. Preferably the yellow pigment is a metal oxide. Suitable metal oxides include titanium dioxide, iron oxide, or stannous oxide. Alternately the yellow pigment is of organic origin. Commercially available yellow pigments include BGYO-TTB2 (from Kobo Product Inc, USA), SI2 Yellow LLXLO (from Daito Kasei Kogyo Company, Japan), Bronze 43737 (from Sudarshan Chemicals, India), Tatrazine Yellow (from Davarson, India), turmeric extract or a mixture thereof. Most preferred is iron oxide with the Colour Index Constitution Number (C.I.) 77492.

The preferred metal oxide is yellow iron oxide. Preferably the iron oxide is coated or encapsulated to minimize interactions with other ingredients of the composition. For example, when organic sunscreens are present in the formulation e.g., butylmethoxydibenzoylmethane sold as Parsol® 1789 (Givaudan), they may interact with iron oxide and produce an undesirable red colour. To minimize such interactions, the metal oxide is coated with materials like mica and/or silicones. An example of a coated yellow iron oxide is BGYO-TTB2 which has I.N.C.I Name: Iron Oxides (C.I. 77492) (And) Isopropyl Titanium Triisostearate (And) Triethoxysilylethyl Polydimethylsiloxyethyl Dimethicone.

Compositions of the present invention preferably also comprise a cosmetically acceptable carrier. Preferably the carrier comprises a significant amount of the fluid having refractive index ($n_c$) of at least 1.0.

Accordingly, preferably the carrier comprises the volatile liquid or the non volatile liquid. In one aspect the carrier is a water-in-oil emulsion. Alternatively the carrier is an oil-in-water emulsion. Where the carrier is an emulsion of either description, it is preferred that the particulate material is pre-dispersed in the continuous phase to minimize interaction of the particle active with any liquid which may be present in the composition.

Preferably the carrier comprises silicones.

Silicones may be divided into the volatile and nonvolatile variety. Volatile silicone oils (if used) are preferably chosen from cyclic (cyclomethicone) or linear polydimethylsiloxanes containing from 3 to 9, preferably from 4 to 5, silicon atoms.

Nonvolatile silicones useful as an emollient include polyalkyl siloxanes, polyalkylaryl siloxanes and polyether siloxane copolymers. The essentially nonvolatile polyalkyl siloxanes useful herein include, for example, polydimethyl siloxanes with viscosities of from about $5 \times 10^{-6}$ to $0.1\ m^2/s$ at 25 °C. Among the preferred nonvolatile emollients useful in the present compositions are the polydimethyl siloxanes having viscosities from about $1 \times 10^{-s}$ to about $4 \times 10^{-4}\ m^2/s$ at 25 °C.

Specific examples of non-silicone emollients include stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n- butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate, and mixtures thereof.

Among the ester emollients are:

(a) alkenyl or alkyl esters of fatty acids having 10 to 20 carbon atoms. Examples thereof include isoarachidyl neopentanoate, isodecyl neopentanoate, isononyl isonanoate, cetyl ricinoleate, oleyl myristate, oleyl stearate, and oleyl oleate;

(b) ether-esters such as fatty acid esters of ethoxylated fatty alcohols;

(c) polyhydric alcohol esters. Butylene glycol, ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of $C_1$-$C_{30}$ alcohols. Exemplative is pentaerythrityl tetraethylhexanoate;

(d) wax esters such as beeswax, spermaceti wax and tribehenin wax;

(e) sterols esters, of which cholesterol fatty acid esters are examples thereof;

(f) sugar ester of fatty acids such as sucrose polybehenate and sucrose polycottonseedate; or

(g) mixtures of two or more of the foregoing (a) to (f).

Of particular use also are the $C_{12-15}$ alkyl benzoate esters sold under the Finsolve® brand.

Hydrocarbons which are suitable emollients include petrolatum, mineral oil, $C_{11}$-$C_{13}$ isoparaffins, polyalphaolefins, isohexadecane or a mixture thereof.

Alternatively the carrier comprises 1 to 25 wt% fatty acid or 0.1 to 80 wt% soap by weight of the composition. Mixtures of fatty acid and soap are also suitable e.g., forming a vanishing cream base which lends a matte feel to the skin. $C_{12-20}$ fatty acids are especially preferred, more preferred being $C_{14-18}$ fatty acids. The most preferred fatty acid is stearic acid, myristic acid or a mixture thereof. When present, the composition comprises 5 to 20 wt% of the fatty acids or soap. Soaps in the hydrophobic material can include alkali metal salt of fatty acids, like sodium or potassium salts, most preferred being potassium stearate. Generally a vanishing cream base in cosmetic compositions is prepared by taking a desired amount of total fatty matter and mixing with potassium hydroxide in desired amounts. The soap is usually formed in-situ during the mixing.

Preferably the carrier comprises water. Amounts of water may, for example, range from 1 to 85 wt%, more preferably from 5 to 90%, even more preferably from 35 to 80%, optimally between 40 and 70% by weight of the cosmetic composition, depending on the nature of the composition.

Further preferably the compositions of the invention comprise a skin lightening agent. The skin lightening agent is preferably chosen from one or more of a vitamin B3 compound or its derivative e.g. niacin, nicotinic acid or niacinamide or other well known skin lightening agents e.g. adapalene, aloe extract, ammonium lactate, anethole derivatives, apple extract, arbutin, azelaic acid, kojic acid, bamboo extract, bearberry extract, bletilla tuber, bupleurum falcatum extract, burnet extract, butyl hydroxy anisole, butyl hydroxy toluene, citrate esters, Chuanxiong, Dang-Gui, deoxyarbutin, 1,3-diphenyl propane derivatives, 2,5-dihydroxybenzoic acid and its derivatives, 2-(4-acetoxyphenyl)-1,3-dithane, 2-(4- hydroxyphenyl)-1,3-dithane, ellagic acid, escinol, estragole derivatives, Fadeout (Pentapharm), Fangfeng, fennel extract, ganoderma extract, gaoben, Gatuline Whitening (Gattlefosse), genistic acid and its derivatives, glabridin and its derivatives, gluco pyranosyl-1-ascorbate, gluconic acid, glycolic acid, green tea extract, 4-hydroxy-5-methyl- 3[2H]-furanone, hydroquinone, 4-hydroxyanisole and its derivatives, 4-hydroxy benzoic acid derivatives, hydroxycaprylic acid, inositol ascorbate, lemon extract, linoleic acid, magnesium ascorbyl phosphate, Melawhite (Pentapharm), morus alba extract, mulberry root extract, 5-octanoyl salicylic acid, parsley extract, phellinus linteus extract, pyrogallol derivatives, 2,4-resorcinol derivatives, 3,5-resorcinol derivatives, rose fruit extract, salicylic acid, Song-Yi extract, 3,4,5-trihydroxybenzyl derivatives, tranexamic acid, vitamins like vitamin B6, vitamin B12, vitamin C, vitamin A, dicarboxylic acids, resorcinol derivatives, extracts from plants viz. rubia and symplocos, hydroxycarboxylic acids like lactic acid and their salts e.g. sodium lactate, and mixtures thereof. Vitamin B3 compound or its derivative e.g. niacin, nicotinic acid or niacinamide are the more preferred skin lightening agent as per the invention, most preferred being niacinamide. Niacinamide, when used, is preferably 0.1 to 10 wt%, more preferably 0.2 to 5 wt%.

The compositions may further preferably comprise one or more organic sunscreens. A wide variety of organic sunscreen agents are suitable for use in combination with the essential ingredients of this invention. Suitable UV-A / UV-B sunscreen agents include, 2- hydroxy-4-methoxybenzophenone, octyldimethyl p-aminobenzoic acid, digalloyltrioleate, 2,2-dihydroxy-4-methoxybenzophenone, ethyl-4-(bis(hydroxypropyl)) aminobenzoate, 2-ethylhexyl-2-cyano-3,3-diphenylacrylate, 2-ethylhexylsalicylate, glyceryl p-aminobenzoate, 3,3,5-trimethylcyclohexylsalicylate, methylanthranilate, p-dimethyl- aminobenzoic acid or aminobenzoate, 2-ethylhexyl-p-dimethyl- aminobenzoate, 2-phenylbenzimidazole-5-sulfonic acid, 2-(p-dimethylaminophenyl)-5-sulfonicbenzoxazoic acid, 2-ethylhexyl-p-methoxycinnamate, butylmethoxydibenzoylmethane, 2-hydroxy-4- methoxybenzophenone, octyldimethyl-p-aminobenzoic acid and mixtures thereof. The most suitable organic sunscreens are 2-ethylhexyl-p-methoxycinnamate, butylmethoxydibenzoylmethane or a mixture thereof.

A safe and effective amount of organic sunscreens is 0.1 to 10 wt%, more preferably from 0.1 to 5 wt%, of organic sunscreen agent.

Other materials which can be included in the cosmetically acceptable carrier include humectants, thickeners and powders. Examples of each of these types of material, which can be used singly or as mixtures, are as follows:

Humectants include those of the polyhydric alcohol-type. Typical polyhydric alcohols include polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, isoprene glycol, 1,2,6-hexanetriol, glycerol, ethoxylated glycerol, propoxylated glycerol and mixtures thereof. The amount of humectant may range, for example, anywhere from 0.5 to 50%, more preferably between 1 and 15% by weight of the composition. Most preferred is glycerol (also known as glycerin). Amounts of glycerin may range, for example, from 0.5% to 50%, more preferably from 1 to 35%, optimally from 2 to 15% by weight of the composition.

A variety of thickening agents may be included in the compositions. Illustrative but not limiting are stearic acid, Acrylamide/Sodium Acryloyldimethyltaurate Copolymer (Aristoflex® AVC), Hydroxyethyl Acrylate/Sodium Acryloyldimethyltaurate Copolymer, Aluminum Starch Octenyl Succinate, Polyacrylates (such as Carbomers including Carbopol® 980, Carbopol® 1342, Pemulen TR-2® and the Ultrez® thickeners), Polysaccharides (including xanthan gum, guar gum, pectin, carageenan and sclerotium gums), celluloses (including carboxymethyl cellulose, ethyl cellulose, hydroxyethyl cellulose and methyl hydroxymethyl cellulose), minerals (including talc, silica, alumina, mica and clays, the latter being represented by bentonites, hectorites and attapulgites), magnesium aluminum silicate and mixtures thereof. Amounts of the thickeners may range, for example, from 0.05 to 10 wt%, more preferably from 0.3 to 2 wt% by weight of the composition.

Powders include chalk, talc, Fuller's earth, kaolin, starch, gums, colloidal silica sodium polyacrylate, tetraalkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose and ethylene glycol monostearate.

The composition can be formulated in any known format, more preferred formats being creams and lotions. The cosmetic compositions of the invention may further comprise other ingredients which are common in the art to enhance physical properties and performances. Suitable ingredients include but are not limited to binders, colorants and pigments, pH adjusting agents, preservatives, optics, perfumes, viscosity modifiers, biological additives, buffering agents, conditioners, natural extracts, essential oils and skin benefit agents including anti-inflammatory agents, cooling agents, antiperspirant agents, anti-aging agents, anti-acne agents, anti-microbial agents and antioxidants.

The cosmetic composition of this invention is a composition suitable for topical application to human skin, including leave-on and wash-off products. It is preferred that the compositions of the invention are leave-on compositions.

The compositions may be suitable packed in an appropriately sized packaging or dispenser. Packaging can be a jar or tube as well as any other format typically seen for cosmetic, cream, washing and lotion type products. The compositions may be applied topically and preferably 1 to 4 milligrams of composition is applied per square centimeter of skin.

Method and Use

In a second aspect is disclosed a method of blurring surface imperfections of skin comprising a step of applying thereon a cosmetic composition of the first aspect.

The method is non-therapeutic in nature. Non-therapeutic nature means for cosmetic purposes.

In a third aspect is disclosed use of a composition of the first aspect for blurring surface imperfections of skin. Examples include reducing fine lines, wrinkles, pores and/or blemish spots; evening the tone of the skin, or a combination thereof.

In a fourth aspect is disclosed a method of lightening the tone of skin comprising a step of applying thereon a cosmetic composition of the first aspect.

In a fifth aspect is disclosed use of a cosmetic composition of the first aspect for lightening the tone of skin.

The following examples are provided to facilitate an understanding of the invention. The examples are not intended to limit the scope of the claims.

**Examples**

The core-shell particulate material was prepared as follows:
This example demonstrates the manufacture of particulate material useful for the invention and wherein the shell is formed from sol-gel polymerized inorganic polymer.

Materials

Styrene (St) was purchased from Sinopharm Chemical Reagent Co. (China) and purified by treating with 5 wt % aqueous NaOH to remove the inhibitor. An MTC [2-(methacryloyl)-ethyltrimethylammonium chloride] (80 wt %) aqueous solution was supplied by SIGMA-ALDRICH, Co (USA). 2,2'-Azoisobutyronitrile (AIBN, 98%, Aldrich USA) was recrystallized from tetrahydrofuran (THF) before use. a,a'-azodiisobutyramidine dihydrochloride (AIBA), ploy(vinyl pyrrolidone) (PVP, K30, MW was 30 000 D), TEOS (triethylorthosilicate), absolute ethanol, and aqueous ammonia solution (28 wt %) were purchased from Sinopharm Chemical Reagent Co.(China) and used as received. Deionized water was applied for all polymerization and treatment processes.

Synthesis of PS Templates

Polystyrene (PS) particles with particle size above 1 $\mu$m were synthesized by dispersion polymerization. In a typical procedure, positively charged PS particles with a diameter of 1500 nm were synthesized as follows: 10.0 g of Styrene, 3.0 g of PVP, 0.35 g of AIBN, 40.0 g of ethanol, 10.0 g of water were mixed in a 500 ml three-necked flask equipped with a mechanical stirrer, thermometer with a temperature controller, a nitrogen inlet, a Graham condenser, and a heating mantle. The mixing solution was first deoxygenated by bubbling nitrogen gas at room temperature for 30 minutes, and then heated to 70 °C with a stirring rate of 180 rpm for 1.5 hours, followed by addition of a mixture of 10.0 g Styrene, 40.0 g of ethanol and 0.78 g of MTC.

Table 1 summarizes recipes for the synthesis of different sized PS template spheres (Styrene 20 g, PVP 3.0 g, AIBN 0.35 g).

Table 1

| $H_2O$ (g) | EtOH (g) | EtOH(g)/$H_2O$ (g) | Particle size (nm) |
|---|---|---|---|
| 15 | 75 | 1:5 | 1000 |
| 10 | 80 | 1:8 | 1500 |
| 7 | 83 | 1:11.8 | 1800 |

Synthesis of SiO$_2$ Hollow Spheres

In a typical run, 5.5 ml of ammonia was added to the above-obtained dispersion and the mixture was stirred at 180 rpm for 5 minutes. Fifteen g of TEOS was added quickly and the mixture was maintained at 50 °C. for around 1 hour under constant stirring. The obtained spheres were separated from the reaction solution by centrifuging at 10000 rpm for 7 minutes. PS/SiO$_2$ hybrid particles were dried at 80 °C overnight to yield dried powders.

The calcination step used for removing PS core template was performed as the follows:

The products were heated from room temperature to 550 °C at a heating rate of 1 °C/minute in the air and then kept at 550 °C for another 2 hours. Finally, the products were cooled down to room temperature.

SEM images of the SiO.sub.2 hollow spheres obtained with different diameters showed that their diameters roughly equated with the diameters of the PS templates (i.e., 1 μm nm, 1.5 μm and 1.8 μm) and that the shell of each particle had a thickness of around 20 nm.

Details of some other ingredients used in the formulations/compositions in this Section are tabulated in Table 2.

Table 2

| Trade name | INCI | Supplier | Mean Size (μm) | Oil Absorption (Linseed oil g/ 100g) |
|---|---|---|---|---|
| MSS-500 /3H® | Silica (Porous) | Kobo | 3 | 305 |
| Particulate material as prepared above | -- | -- | 1 | 1020 |
| DC 9509® (63% solids content) | Dimethicone/Vinyldimethicone Crosspolymer (and) C12-14 Pareth-12 | Dow Corning | 3 | - |
| Sunspheres® | Styrene/Acrylates Copolymer | Dow Chemicals | 0.4 | 136 |

The formulations of various compositions that were prepared and tested are disclosed in Table 3. Some of the compositions were outside the scope of the present invention and reference thereto is also made in the Table.

Table 3

| Phase | Details of Ingredients | Reference Code<br>Wt% of the Ingredient | | | | | | | | |
|---|---|---|---|---|---|---|---|---|---|---|
| | | A | B | C | D | E | F | G | H | I |
| B | Particulate material | 1 | 1 | 1 | 3 | 0 | 0 | 0 | 0 | 1 |
| | Sunspheres® | 0 | 0 | 0 | 0 | 0 | 0 | 0 | 1 | 0 |
| | MS-500®/3H® | 0 | 0 | 0 | 0 | 1 | 0 | 0 | 0 | 0 |
| A | VP/VA copolymer | 2 | 2 | 2 | 2 | 2 | 2 | 2 | 2 | 2 |
| | Tween® 20 | 1 | 1 | 1 | 1 | 1 | 1 | 1 | 1 | 1 |
| | Glycerin | 2 | 2 | 2 | 2 | 2 | 2 | 2 | 2 | 2 |
| | DC 9509® | 0 | 5 | 2 | 2 | 5 | 16 | 38 | 5 | 5 |
| | Water | Balance to 100 wt% of the composition | | | | | | | | |
| C | Isopropyl myristate | 12 | 12 | 12 | 12 | 12 | 12 | 12 | 12 | 0 |
| | DC 245 | 0 | 0 | 0 | 0 | 0 | 0 | 0 | 0 | 12 |
| D | SimulGel® EG | 3 | 3 | 3 | 3 | 3 | 3 | 3 | 3 | 3 |
| E | Preservative | 0.5 | 0.5 | 0.5 | 0.5 | 0.5 | 0.5 | 0.5 | 0.5 | 0.5 |

Note: Composition D is within the invention

Process:

The Phase A ingredients are put in a mixer of suitable size. Thereafter, Phase B ingredients are added into the mixer under stirring, and later with homogenizer until well dispersion is complete. This is followed by addition of half the total quantity of the ingredients of Phase D. Thereafter, the Phase C ingredient is added along with balance amount of the Phase D ingredients into the main mixer under homogenizer. When the Phase C ingredient is added, the ingredient gets inside the hollow core of the particulate material and it eventually becomes the core of the material and remains there until the composition is applied to the skin. Finally, the Phase E ingredient is added to get cosmetic compositions in the form of creams.

Test methods:

The test methods relevant to the compositions of the present invention are briefly summarized herein.

A) L&W Index (Blurring Efficacy)

This method has two steps. In the first step, the gloss of artificial skin is measured before application of any cosmetic composition.
Wrinkled Bio-Skin Plates (BP-EW1 #BSC, Beaulax Co., Ltd., Tokyo, Japan) made of polyurethane elastomer were used to mimic wrinkled human skin. Hereinafter they are abbreviated as BSP. A dual-polarized image system called SAMBA (Bossa Nova Technologies, USA) was employed to measure the gloss of the wrinkled Bio-skin plates by following the method and principle described by Matsubara et.al., [Skin Translucency: What Is It & How Is It Measured, The International Federation of Societies of Cosmetic Chemists (IFSCC) Congress 2006, Osaka, Japan]. A software named SAMBA face system (Version 4.3) was equipped for the analysis. The Wrinkled Bio-skin plates were tested against an incident light with exposure time of 80 mili seconds. The operation modes were parallel polarization and crossed polarization modes. Thereafter, 28 mg of the concerned cream was applied thereon and it was spread by finger cot within the circle of 7 cm$^2$. The sample was left to dry for 30 minutes before being subjected to any further testing. The gloss was measured again as done before.
In the second step, the L&W Index was determined as follows. This Index is directly proportional to the blurring efficacy.
The incident light was reflected and scattered by Bio-skin plates. The specular reflected light kept the same polarization as the incident light whereas the scattering light from the volume (diffused light) was un-polarized. The camera successively acquires two images corresponding to two states of polarization (parallel and crossed). The parallel image intensity (P) is contributed to by the reflected and the scattered light, and the crossed image intensity (C) is contributed to by the scattered light. The parallel image plus the crossed image is equal to the total image delivered by a traditional camera or perceived by human eye. The degree of gloss is as follows, (P-C)/(P+C). The calculation of gloss degree was performed for each pixel. The standard deviation (STD) of gloss degree is a measure of the uniformity of the skin appearance. The higher the STD is, the lower the uniformity is. Herein we defined a L&W (line and wrinkle) index to demonstrate degree of blurring efficacy of the skin care composition. The L&W index was calculated by (STD of gloss degree before applying sample - STD of gloss degree after applying sample) / (STD of gloss degree before applying sample). The L&W Index is directly proportional to the blurring efficacy of the composition.

B) Deposition of oil to simulate secretion of sebum

Human skin tends to secrete sebum and over time it accumulates on the skin. Such accumulated sebum has the potential to affect the performance of cosmetic compositions. Therefore, it is necessary to test the compositions by simulating the secretion of sebum on skin. This was done with the help of humidifiers to generate fine oil droplets which were allowed to deposit on the surface of BSP. The wrinkled BSP were left in the chamber where humidifiers were placed for accumulation of oil. The extent of deposition was measured by weighing the plates. The L&W index was measured after an average of 6 mg oil had deposited on every $cm^2$ of the plate.

The observations are tabulated in Tables 4 and 5.

Table 4: L* values indicative of whitening

| Composition Reference number | ΔL* on 20# BSP | ΔL* on 50# BSP |
|---|---|---|
| A of Table 2 | 0.96 ± 0.17 | 1.96 ± 0.38 |
| B of Table 2 | 0.31 ± 0.21 | 1 ± 0.22 |
| C of Table 2 | 0.88 ± 0.22 | 0.71 ± 0.34 |
| D of Table 2 | 8.47 ± 0.87 | 11.56 ± 2.09 |

Note:

(1) 20# BSP indicates
(2) 50# BSP indicates
(3) ΔL* is the difference between L* of sample and control (no sample) plates and it is directly proportional to the whitening effect

L* value observed in the case of composition A indicates that inclusion of the core-shell particulate material leads to an increased whiteness index. On comparison with the data observed in the case of composition B (ratio of the amount of the particulate material to that of the silicone elastomer is 1:5), it is evident that the further addition of silicone elastomer decreases the whitening effect. Therefore, the composition B is out of the scope of the present invention. However, when the ratio between the two ingredients is altered as in compositions C (ratio of the amount of the particulate material to that of the silicone elastomer is 1:2) and D (ratio of the amount of the particulate material to that of the silicone elastomer is 3:2), it is evident that compositions C and D provide whitening effect.

Therefore, at the desire of the formulator, the compositions may be altered by adjusting the ratio of the two ingredients to get whitening effect or lesser whitening effect, depending on the said ratio.

Table 5

| Composition Ref No of Table 3 | L&W index | |
| | After application | After 6 mg oil loading |
|---|---|---|
| A | 34.41% ± 9.0% | -39.91% ± 11.6% |
| B | 40.3% ± 5.6% | 42.0% ± 6.0% |
| C | 35.4% ± 0.2% | 38.6% ± 4.0% |
| D | 40.3% ± 5.6% | 42.0% ± 6.0% |
| E | 6.5% ± 0.3% | - 81.1% ± 21.2% |
| F | 12.4% ± 3.9% | -- |

(continued)

| Composition Ref No of Table 3 | L&W index | |
| --- | --- | --- |
| | After application | After 6 mg oil loading |
| G | 36.1% ± 4.7% | -- |
| H | - 27.2% ± 4.8% | - 75.7% ± 6.8% |
| I | 36.43% ± 9.2% | 41.43% ± 6.8% |

Note:

The L/W Index is directly proportional to the efficacy of the composition to cause a blurring effect. The Index is expected to fall/decrease on sebum-rich skin but an efficacious composition will resist the decrease.

On comparing the data pertaining to composition B with E, it is evident that the efficacy of B is significantly greater than E, not just on application but even after loading the oil. MSS-500/3H® is spherical shaped white powder, 3 μm in size, composed of Silica, and used as a Silica Microsphere. The microspheres are said to offer a ball-bearing effect to impart finished products with an elegant silky texture, increased payoff, and enhanced slip which promotes better blend ability on the skin. They are also said to be able to scatter light to diminish the look of fine lines on the skin, while letting enough light through so the look of the skin is natural. However, the performance of composition B is far better.

Comparison between compositions B and H indicate that inorganic core-shell particulate matter is better than its organic counterpart. SUNSPHERES is a styrene/acrylates copolymer manufactured via emulsion polymerization.

The data pertaining to compositions F and G indicate that the known blurring active, i.e., DC 9509® which is a silicone elastomer, is highly effective when it comes to the L&W Index. The problem, however, is that the concerned compositions have as much as 16 and 38 wt% (63% solids content) of the elastomer, which is substantially high loading. On the other hand, compositions C and D provide equivalent or higher instant blurring effect while allowing or permitting significant reduction in the amount of the elastomer. The data pertaining to compositions B, C and D further indicates little, or practically no variation in the L/W Index upon alteration of the ratio between the core-shell particulate material and the silicone elastomer.

The data pertaining to compositions B and I indicate that both volatile and non-volatile fluid (emollient) deliver good efficacy on application and which sustains itself even after application of oil.

**Claims**

1.    A cosmetic composition comprising:

(a) a particulate material comprising a shell and a core, where said shell comprises a water-insoluble material having refractive index ($n_s$) of 1.2 to 2.0 at 25 °C and wavelength of 589 nm and core comprises a fluid having refractive index ($n_c$) of at least 1.0 at 25 °C and wavelength of 589 nm and where oil absorption capacity of said particulate material is at least 300 g/100 g measured by ASTM D281-95 by Spatula Rub-out using linseed oil ; and,
(b) a particulate silicone elastomer having average particle diameter of 0.1 to 100 μm,

wherein ratio of the amount of said particulate material to that of said particulate silicone elastomer is 1:3.5 to 3.5:1 parts by weight,
and wherein the composition comprises 2.5 to 10 wt% of the particulate material.

2.    A cosmetic composition as claimed in claim 1 wherein particles of said particulate silicone elastomer are spherical or spheroidal.

3.    A cosmetic composition as claimed in any of claims 1 or 2 wherein particle size of said particulate material is 0.1 to 2 μm.

4.    A cosmetic composition as claimed in any of claims 1 to 3 wherein said silicone elastomer is a vinyldimethicone crosspolymer.

5.    A cosmetic composition as claimed in any of claims 1 to 4 comprising 0.1 to 10 wt% of said particulate material.

**6.** A cosmetic composition as claimed in any of claims 1 to 5 comprising 0.1 to 10 wt% of said silicone elastomer.

**7.** A cosmetic composition as claimed in any of claims 1 or 6 wherein said fluid is a volatile liquid or a non-volatile liquid.

**8.** A cosmetic composition as claimed in claim 7 wherein said volatile liquid is water, cyclomethicone or a cosmetically acceptable volatile solvent other than water.

**9.** A cosmetic composition as claimed in any of claims 1 to 8 wherein said water-insoluble material having refractive index ($n_s$) of 1.2 to 2.0 at 25 °C and wavelength of 589 nm is an inorganic material.

**10.** A cosmetic composition as claimed in claim 9 wherein said inorganic material is an inorganic polymer or an inorganic polymer comprising one or more organic groups.

**11.** A cosmetic composition as claimed in claim 10 wherein said inorganic polymer comprises silica or silica modified with one or more organic groups.

**12.** A cosmetic composition as claimed in any of claims 1 to 11 wherein ratio of refractive index of the core to that of the shell ($n_c/n_s$) is from 0.7 to 1.2 at 25 °C and wavelength of 589 nm.

**13.** A method of blurring surface imperfections of skin comprising a step of applying thereon a cosmetic composition as claimed in any of claims 1 to 12.

**14.** Use of a cosmetic composition as claimed in any of claims 1 to 12 for blurring surface imperfections of skin.

**Patentansprüche**

**1.** Kosmetische Zusammensetzung, umfassend:

(a) ein teilchenförmiges Material, umfassend eine Schale und einen Kern, wobei die Schale ein wasserunlösliches Material mit einem Brechungsindex ($n_s$) von 1,2 bis 2,0 bei 25 °C und einer Wellenlänge von 589 nm umfasst und der Kern ein Fluid mit einem Brechungsindex ($n_c$) von mindestens 1,0 bei 25 °C und einer Wellenlänge von 589 nm umfasst, und wobei das Ölabsorptionsvermögen des teilchenförmigen Materials mindestens 300 g/100 g beträgt, gemessen gemäßASTM D281-95durch Spatel Rub-out unter Verwendung von Leinsamenöl; und
(b) ein teilchenförmiges Siliconelastomer mit einem mittleren Teilchendurchmesser von 0,1 bis 100 $\mu$m,

wobei das Verhältnis der Menge des teilchenförmigen Materials zu der des teilchenförmigen Siliconelastomers 1:3,5 bis 3,5:1 Gewichtsteile beträgt, und wobei die Zusammensetzung 2,5 bis 10 Gew.-% des teilchenförmigen Materials umfasst.

**2.** Kosmetische Zusammensetzung wie in Anspruch 1 beansprucht, wobei Teilchen des teilchenförmigen Siliconelastomers sphärisch oder sphäroidal sind.

**3.** Kosmetische Zusammensetzung wie in irgendeinem der Ansprüche 1 oder 2 beansprucht, wobei die Teilchengröße des teilchenförmigen Materials 0,1 bis 2 $\mu$m beträgt.

**4.** Kosmetische Zusammensetzung wie in irgendeinem der Ansprüche 1 bis 3 beansprucht, wobei das Siliconelastomer ein Vinyldimethicon-Kreuzpolymer ist.

**5.** Kosmetische Zusammensetzung wie in irgendeinem der Ansprüche 1 bis 4 beansprucht, umfassend 0,1 bis 10 Gew.-% des teilchenförmigen Materials.

**6.** Kosmetische Zusammensetzung wie in irgendeinem der Ansprüche 1 bis 5 beansprucht, umfassend 0,1 bis 10 Gew.-% des Siliconelastomers.

**7.** Kosmetische Zusammensetzung wie in irgendeinem der Ansprüche 1 oder 6 beansprucht, wobei das Fluid eine flüchtige Flüssigkeit oder eine nichtflüchtige Flüssigkeit ist.

**EP 3 716 935 B1**

8. Kosmetische Zusammensetzung wie in Anspruch 7 beansprucht, wobei die flüchtige Flüssigkeit Wasser, Cyclomethicon oder ein kosmetisch akzeptables flüchtiges Lösungsmittel ist, das von Wasser verschieden ist.

9. Kosmetische Zusammensetzung wie in irgendeinem der Ansprüche 1 bis 8 beansprucht, wobei das wasserunlösliche Material mit einem Brechungsindex ($n_s$) von 1,2 bis 2,0 bei 25 °C und einer Wellenlängevon 589 nm ein anorganisches Material ist.

10. Kosmetische Zusammensetzung wie in Anspruch 9 beansprucht, wobei das anorganische Material ein anorganisches Polymer oder ein anorganisches Polymer, das eine oder mehrere organische Gruppen umfasst, ist.

11. Kosmetische Zusammensetzung wie in Anspruch 10 beansprucht, wobei das anorganische Polymer Siliciumdioxid oder Siliciumdioxid, das mit einer oder mehreren organischen Gruppen modifiziert ist, umfasst.

12. Kosmetische Zusammensetzung wie in irgendeinem der Ansprüche 1 bis 11 beansprucht, wobei das Verhältnis von Brechungsindex des Kerns zu dem der Schale ($n_c/n_s$) 0,7 bis 1,2 bei 25 °C und einer Wellenlängevon 589 nm beträgt.

13. Verfahren zum Verundeutlichen von Oberflächenfehlern der Haut, umfassend einen Schritt des Aufbringens einer kosmetischen Zusammensetzung, wie in irgendeinem der Ansprüche 1 bis 12 beansprucht, darauf.

14. Verwendung einer kosmetischen Zusammensetzung wie in irgendeinem der Ansprüche 1 bis 12 beansprucht zum Verundeutlichen von Oberflächenfehlern der Haut.

**Revendications**

1. Composition cosmétique comprenant :

(a) un matériau particulaire comprenant une enveloppe et un noyau, où ladite enveloppe comprend un matériau insoluble dans l'eau ayant un indice de réfraction ($n_s$) de 1,2 à 2,0 à 25°C et une longueur d'onde de 589 nm et le noyau comprend un fluide ayant un indice de réfraction ($n_c$) d'au moins 1,0 à 25°C et une longueur d'onde de 589 nm et où la capacité d'absorption d'huile dudit matériau particulaire est d'au moins 300 g/100 g mesurée par ASTM D281-95 par gommage à la spatule utilisant de l'huile de lin ; et
(b) un élastomère de silicone particulaire ayant un diamètre moyen de particule de 0,1 à 100 $\mu$m,

dans laquelle le rapport de la quantité dudit matériau particulaire à celle dudit élastomère de silicone particulaire est de 1:3,5 à 3,5:1 parties en masse,
et dans laquelle la composition comprend de 2,5 à 10 % en masse du matériau particulaire.

2. Composition cosmétique selon la revendication 1, dans laquelle les particules dudit élastomère de silicone particulaire sont sphériques ou sphéroïdales.

3. Composition cosmétique selon l'une quelconque des revendications 1 ou 2, dans laquelle la taille de particules dudit matériau particulaire est de 0,1 à 2 $\mu$m.

4. Composition cosmétique selon l'une quelconque des revendications 1 à 3, dans laquelle ledit élastomère de silicone est un polymère croisé de vinyldiméthicone.

5. Composition cosmétique selon l'une quelconque des revendications 1 à 4 comprenant de 0,1 à 10 % en masse dudit matériau particulaire.

6. Composition cosmétique selon l'une quelconque des revendications 1 à 5 comprenant de 0,1 à 10 % en masse dudit élastomère de silicone.

7. Composition cosmétique selon l'une quelconque des revendications 1 ou 6, dans laquelle ledit fluide est un liquide volatil ou un liquide non-volatil.

8. Composition cosmétique selon la revendication 7, dans laquelle ledit liquide volatil est l'eau, la cyclométhicone ou

**15**

un solvant volatil cosmétiquement acceptable différent de l'eau.

9. Composition cosmétique selon l'une quelconque des revendications 1 à 8, dans laquelle ledit matériau insoluble dans l'eau ayant l'indice de réfraction ($n_s$) de 1,2 à 2,0 à 25°C et une longueur d'onde de 589 nm est un matériau inorganique.

10. Composition cosmétique selon la revendication 9, dans laquelle ledit matériau inorganique est un polymère inorganique ou un polymère inorganique comprenant un ou plusieurs groupes organiques.

11. Composition cosmétique selon la revendication 10, dans laquelle ledit polymère inorganique comprend de la silice ou de la silice modifiée avec un ou plusieurs groupes organiques.

12. Composition cosmétique selon l'une quelconque des revendications 1 à 11, dans laquelle le rapport d'indice de réfraction du noyau à celui de l'enveloppe ($n_c/ n_s$) est de 0,7 à 1,2 à 25°C et une longueur d'onde de 589 nm.

13. Procédé pour estomper des imperfections de surface de la peau comprenant une étape d'application sur son dessus d'une composition cosmétique selon l'une quelconque des revendications 1 à 12.

14. Utilisation d'une composition cosmétique selon l'une quelconque des revendications 1 à 12 pour estomper des imperfections de surface de la peau.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 15166459 A1
- WO 08018046 A2
- US 2007179241 A
- WO 15055416 A1
- WO 15055494 A1
- WO 2017071886 A1
- WO 2017220310 A1
- WO 2016152872 A
- US 7758888 B1

**Non-patent literature cited in the description**

- **S. GU et al.** *Journal of Colloid and Interface Science,* 2005, vol. 289, 419-426
- **MATSUBARA.** Skin Translucency: What Is It & How Is It Measured. *The International Federation of Societies of Cosmetic Chemists (IFSCC) Congress,* 2006